(19) Europäisches Patentamt
European Patent Office
Office européen des brevets

(11) Veröffentlichungsnummer: **0 357 545 A2**

(12) # EUROPÄISCHE PATENTANMELDUNG

(21) Anmeldenummer: **89810525.9**

(22) Anmeldetag: **12.07.89**

(51) Int. Cl.[5]: **C 07 D 249/20**
C 07 D 251/24,
C 07 D 401/12, C 08 K 5/19,
C 08 K 5/349, C 08 K 5/347

(30) Priorität: **21.07.88 CH 2794/88**

(43) Veröffentlichungstag der Anmeldung:
**07.03.90 Patentblatt 90/10**

(84) Benannte Vertragsstaaten:
**CH DE ES FR GB IT LI**

(71) Anmelder: **CIBA-GEIGY AG**
**Klybeckstrasse 141**
**CH-4002 Basel (CH)**

(72) Erfinder: **Höhener, Alfred, Dr.**
**Langweg 1**
**CH-4312 Magden (CH)**

**Burdeska, Kurt, Dr.**
**Laufenburgerstrasse 30**
**CH-4058 Basel (CH)**

**Reinert, Gerhard, Dr.**
**Weiherweg 1/7**
**CH-4123 Allschwil (CH)**

Patentansprüche für folgenden Vertragsstaat: ES.

(54) **Kationische Verbindungen, deren Herstellung und deren Anwendung zur fotochemischen Stabilisierung basisch anfärbbarer Polyamid-, Polyacrylnitril-und Polyesterfasermaterialien.**

(57) Neue kationische Verbindungen der Formel

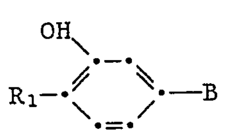

worin X, $Y_1$, $Y_2$, $Y_3$, A, $R_o$ und $R_2$-$R_7$ die in Anspruch 1 angegebene Bedeutung haben, werden zur fotochemischen Stabilisierung von basisch anfärbbaren Polyamid-, Polyacrylnitril- und Polyesterfasermaterialien verwendet. Die Herstellung der neuen Verbindungen sowie der neuen Ausgangsstoffe wird beschrieben.

EP 0 357 545 A2

## Beschreibung

### Kationische Verbindungen, deren Herstellung und deren Anwendung zur fotochemischen Stabilisierung basisch anfärbbarer Polyamid-, Polyacrylnitril- und Polyesterfasermaterialien

Die vorliegende Erfindung betrifft neue kationische Verbindungen, deren Herstellung und deren Anwendung zur fotochemischen Stabilisierung basisch anfärbbarer Polyamid-, Polyacrylnitril- und Polyesterfasermaterialien sowie die zur Herstellung der Endprodukte benötigten neuen Ausgangsstoffe.

Bei "Differential-dyeing"-Teppichen stellt die mangelnde Lichtechtheit des basisch anfärbbaren Polyamidanteils, besonders die Vergrünung der Rotkomponente ein erhebliches Problem dar.

Es wurde nun gefunden, dass man das Problem weitgehend lösen kann, wenn man die Fasermaterialien mit Färbeflotten färbt, die zusätzlich neue kationische Verbindungen enthalten.

Die vorliegende Erfindung betrifft daher neue kationische Verbindungen der Formel

(1) 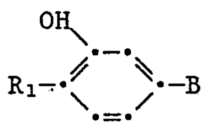

worin B eine Gruppe der Formel

(Ia) 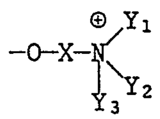 • $A^{\ominus}$ und

$R_1$ einen Rest der Formel

(2) 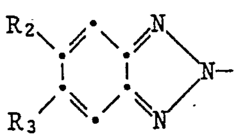 , (3) 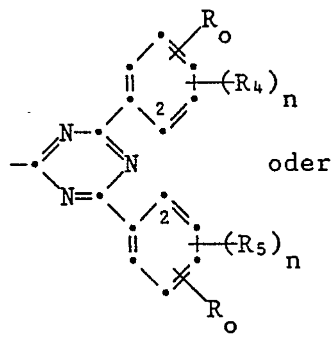 oder (4) 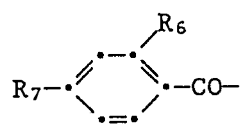

worin

$R_0$ für Wasserstoff oder Hydroxy,

$R_2$ für Wasserstoff, Halogen, $C_1$-$C_4$-Alkyl, $C_1$-$C_4$-Alkoxy, $C_2$-$C_9$-Alkoxycarbonyl oder Carboxy,

$R_3$ für Wasserstoff oder Halogen,

$R_4$ und $R_5$ unabhängig voneinander für Wasserstoff, $C_1$-$C_4$-Alkyl, $C_1$-$C_4$-Alkoxy oder Halogen, oder wenn $R_0$ Hydroxy und n 1 sind auch die Gruppe der Formel (Ia),

$R_6$ für Wasserstoff, Hydroxy oder Carboxy,

$R_7$ für Wasserstoff, Hydroxy oder $C_1$-$C_4$-Alkoxy und

n für die Zahl 1 oder 2

stehen,

X $C_2$-$C_8$-Alkylen,

$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring,

$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,

$Y_3$ Wasserstoff, unsubstituiertes oder durch Cyano, Hydroxy, $C_1$-$C_4$-Alkoxy, Phenyl oder $C_1$-$C_4$-Alkoxycarbonyl substituiertes $C_1$-$C_4$-Alkyl oder $C_3$-$C_4$-Alkenyl,

$Y_1$, $Y_2$ und $Y_3$ zusammen mit dem sie verbindenden N-Atom auch den Pyridin-oder Picolinring und

$A^{\ominus}$ ein farbloses Anion

bedeuten.

Stellt n die Zahl 1 und $R_0$ Hydroxy dar, dann ist der Hydroxyrest in 2-Stellung und die Gruppe (Ia) in 4-Stellung. $R_0$ ist vorzugsweise Wasserstoff.

Als 5- bis 7-gliedrige heterocyclische Ringe, die $Y_1$ und $Y_2$ zusammen mit dem sie bindenden N-Atom bilden können, kommen z.B. ein Morpholin-, Piperidin-, Pyrrolidin- und Hexamethyleniminring (= Hexahydro-1H-aze-

pin) in Betracht.

$Y_1$, $Y_2$ und $Y_3$ sind vorzugsweise unsubstituiertes $C_1$-$C_4$-Alkyl.

Von besonderem Interesse sind Verbindungen der Formel

(5) 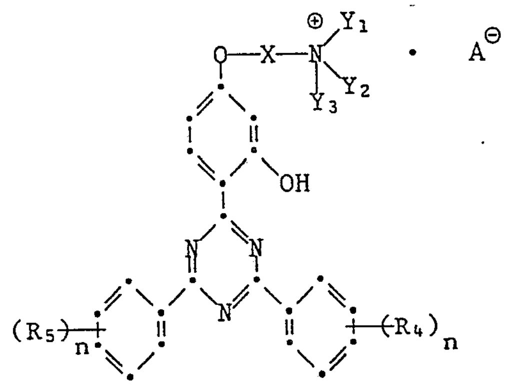

worin $R_4$ und $R_5$ Wasserstoff oder $C_1$-$C_4$-Alkyl, X $C_2$-$C_3$-Alkylen bedeuten und $Y_1$, $Y_2$, $Y_3$, n und $A^{\ominus}$ die unter Formel (1) angegebene Bedeutung haben, sowie der Formel

(6) 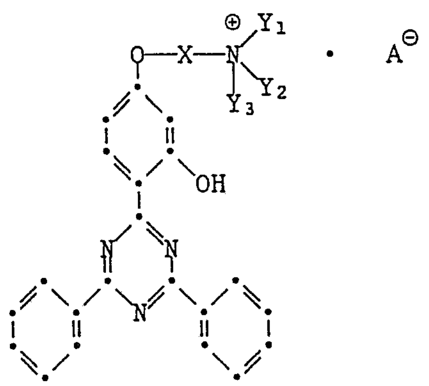

worin X $C_2$-$C_3$-Alkylen,
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

In Verbindungen der Formel (5) sind $R_4$ und $R_5$ vorzugsweise Wasserstoff oder Methyl.

Verbindungen der Formel (1), worin $R_1$ den Rest der Formel (3) ist, in welchem $R_0$ Hydroxy und n die Zahl 1 bedeuten, entsprechen der Formel

(7) 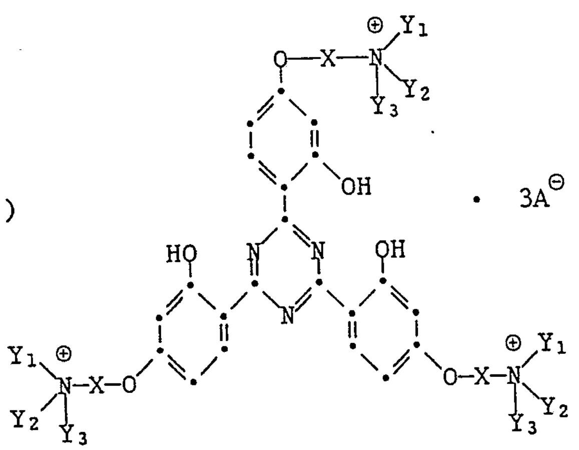

worin X, $Y_1$, $Y_2$, $Y_3$ und $A^{\ominus}$ die unter Formel (1) angegebene Bedeutung haben.

Verbindungen der Formeln (5) und (6) sind bevorzugt.

Ebenfalls von Interesse sind die Verbindungen

a) der Formel

(8) 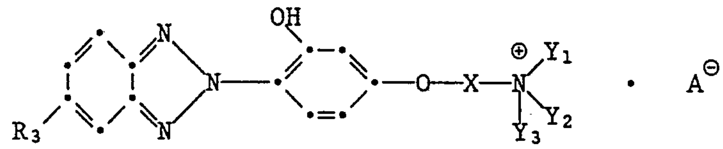

worin
$R_3$ Wasserstoff oder Chlor und
X $C_2$-$C_3$-Alkylen bedeuten und $Y_1$, $Y_2$, $Y_3$ und $A^{\ominus}$ die unter Formel (1) angegebene Bedeutung haben,
b) der Formel

(9) 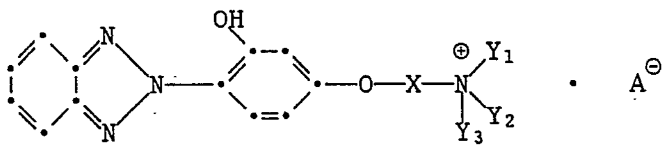

worin
X $C_2$-$C_3$-Alkylen,
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten,
c) der Formel

(10) 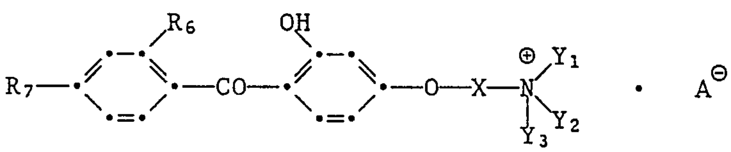

worin
X $C_2$-$C_3$-Alkylen bedeutet und $R_6$, $R_7$, $Y_1$, $Y_2$, $Y_3$ und $A^{\ominus}$ die unter Formel (1) angegebene Bedeutung haben, und
d) der Formel

(11) 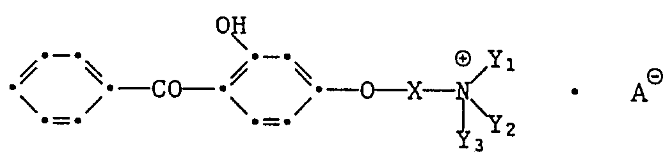

worin
X $C_2$-$C_3$-Alkylen
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

In den vorhergehenden Formeln nehmen die Substituenten folgende Bedeutungen an:
Halogen steht für Fluor, Brom und vorzugsweise Chlor.
$C_1$-$C_4$-Alkyl ist Methyl, Ethyl, n-Propyl, Isopropyl, n-Butyl, Isobutyl, sek. Butyl oder tert. Butyl.
$C_1$-$C_4$-Alkoxy bedeutet Methoxy, Ethoxy, n-Propoxy, Isopropoxy, n-Butoxy, Isobutoxy, sek. Butoxy oder tert. Butoxy.
$C_2$-$C_9$-Alkoxycarbonyl steht für Methoxycarbonyl, Ethoxycarbonyl, n-Propoxycarbonyl, n-Butoxycarbonyl, Pentoxycarbonyl, Hexylcarbonyl, Heptoxycarbonyl oder Octyloxycarbonyl.

Die neuen kationischen Verbindungen der Formel (1) werden dadurch hergestellt, dass man 1 Moläquivalent einer Verbindung der Formel

(12) 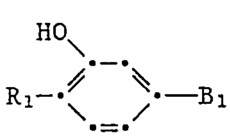

mit einem Moläquivalent eines Alkylierungsmittels bzw. einer Säure der Formel $Y_3$-A bei einer Temperatur von 0 - 180°C quaterniert bzw. protoniert, wobei in diesen Formeln $B_1$ die Gruppe der Formel

(Ib) 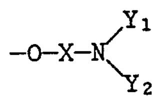

bedeutet und $R_1$, X, $Y_1$, $Y_2$, $Y_3$ und A die unter Formel (1) angegebene Bedeutung haben.

Die Quaternierung bzw. Protonierung wird vorteilhaft bei Temperaturen zwischen 30 und 140°C durchgeführt.

Als Quaternierungs- bzw. Protonierungsmittel $Y_3$-A sind beispielsweise folgende Verbindungen geeignet: Alkylhalogenide wie Methyljodid, Aethyljodid, Aethylbromid, Butylbromid oder Benzylchlorid, Dialkylsulfate wie Dimethyl oder Diäthylsulfat, Sulfonsäureester wie Toluol- oder Benzolsulfonsäuremethyl- oder -äthylester, Alkylenoxide wie Aethylen- oder Propylenoxide oder Eipchlorhydrin, Acrylsäureester wie Acrylsäuremethyl-, -äthyl- oder -butylester, Acrylnitril, die Verbindungen der Formel

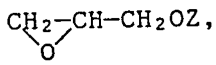

wobei Z für Methyl, Aethyl, Propyl, Butyl oder Phenyl steht. Phosphite oder Phosphonate der Formel

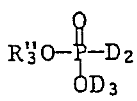

worin R″ Alkyl mit 1 - 4 C-Atomen, $D_2$ Wasserstoff oder unsubstituiertes oder durch Hydroxy, Cyano, Alkylcarbonyloxy oder Alkoxycarbonyl mit jeweils 1 bis 4 C-Atomen im Alkylteil substituiertes Alkyl und $D_3$ Alkyl mit 1 bis 4 C-Atomen bedeuten.

Die Quaternierung der Verbindungen der Formel (12) mit Alkylhalogeniden, Dialkylsulfaten oder Sulfosäureestern zu den Verbindungen der Formel (1) wird zweckmässigerweise in einem gegenüber dem Alkylierungsmittel inerten Lösungsmittel vorgenommen. Geeignete Lösungsmittel sind z.B. Kohlenwasserstoffe wie Benzol, Toluol und Xylol, halogenierte aliphatische oder aromatische Kohlenwasserstoffe wie Chloroform, Aethylenchlorid, Chlorbenzol und Dichlorbenzol, Alkohole wie Aethanol, Butanol, Aethylenglykol und Aethylenglykolmonomethyläther, Aether wie Aethylenglykoldimethyläther und Dioxan oder Amide wie Dimethylformamid und N-Methylpyrrolidon.

Die Quaternierung mit den genannten Alkylierungsmitteln erfolgt vorteilhaft bei Temperaturen zwischen 0 und 180°C, vorzugsweise bei 30 bis 140°C.

Die Quaternierung der Verbindungen der Formel (12) zu den Verbindungen der Formel (1) mit Alkylenoxiden, Epichlorhydrin sowie dessen Derivaten der Formel

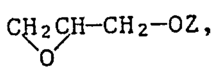

in der Z die genannten Bedeutungen hat, Acrylsäureester oder Acrylnitril wird bei den genannten Temperaturen in saurem Medium, vorteilhaft in Gegenwart einer organischen Säure wie Ameisensäure, Essigsäure, Propionsäure oder Benzoesäure durchgeführt, jedoch können auch anorganische Säuren wie Schwefelsäure, Phosphorsäure oder Halogenwasserstoffsäuren dafür verwendet werden. Diese anorganischen Säuren können in konzentrierter, handelsüblicher Form als verdünnte wässrige Lösungen oder in Mischung mit den genannten organischen Lösungsmitteln, gegebenenfalls unter Zusatz von Wasser, verwendet werden. Erfolgt die Umsetzung in Gegenwart von organischen Säuren, so wird meistens die konzentrierte Form dieser Säuren angewandt, gegebenenfalls in Mischung mit den genannten organischen Lösungsmitteln.

Bevorzugte Phosphite bzw. Phosphonate sind z.B. Dimethylphosphit, Diäthylphosphit, Dimethyl-methanphosphonat, Diäthylmethanphosphonat, Methyl-äthyl-methanphosphonat, Methyl-propyl-methan-phosphonat, Methyl-butyl-methanphosphonat, Methyl-hexyl-methanphosphonat, Methyl-octyl-methanphosphonat, Methyl-decyl-methanphosphonat, Methyl-dodecyl-methanphosphat, Dimethyl-β-hydroxy-äthanphosphonat, Dimethyl-β-acetoxyäthanphosphonat, Dimethyl-β-methoxycarbonyläthan-phosphonat und Dimethyl-β-cyanäthan-phosphonat.

Die Umsetzung erfolgt in Wasser und/oder organischen Lösungsmitteln wie Methanol, Aethanol, Propanol, Isopropanol, Butanol, Glykol, Glykolmethyläther, Glykol-di-methyläther, Glykolbutyläther, Diglykolmethyläther, Methyläthylketon, Methylbutylketon, Dimethylformamid, Sulfolan, Oxypropionitril, Toluol, Xylol, Benzylalkohol, Phenoxyäthanol, Benzyloxypropionitril bei vorzugsweise 60 bis 190°C. Bei der Verwendung von flüssigen Phosphiten oder Phosphonaten kann die Umsetzung auch in Abwesenheit eines zusätzlichen Lösungsmittels vorgenommen werden.

Werden protonierte Verbindungen der Formel (1) gewünscht, d.h. Säure-Additionssalze desselben, so

verwendet man als Protonierungsmittel insbesondere Mineralsäuren. Geeignet sind prinzipiell alle starken bis mittelstarken organischen Säuren oder Mineralsäuren.

Als Lösungsmittel, in denen die Protonierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe wie Benzol, Toluol und Xylol; Halogenkohlenwasserstoffe wie Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Aether wie Butanol, Dibutyläther, Aethylenglykol, Aethylenglykolmonomethyläther, Aethylenglykolmonoäthyläther, Anisol oder Dioxan; Ketone wie Cyclohexanon oder Methyläthylketon; Fettsäureamide wie Dimethylformamid oder Dimethylacetamid; Sulfoxyde wie Dimethylsulfoxyd und Carbonsäureester wie Essigester oder Essigsäurebutylester.

Die Verbindungen der Formel (12) sind ebenfalls neu und stellen daher einen weiteren Gegenstand der vorliegenden Erfindung dar.

Die Verbindungen der Formeln

(13) 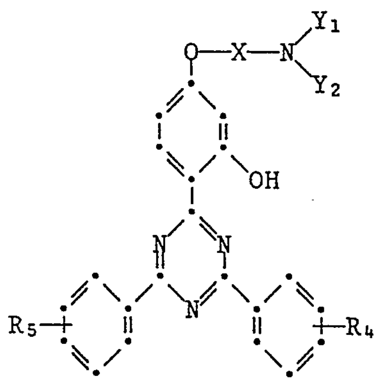

worin $R_4$ und $R_5$ Wasserstoff, $C_1$-$C_4$-Alkyl, $C_1$-$C_4$-Alkoxy oder Halogen, X $C_2$-$C_3$-Alkylen bedeuten und $Y_1$ und $Y_2$ die unter Formel (1) angegebene Bedeutung haben,

(14) 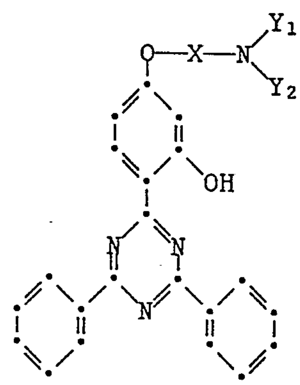

worin X $C_2$-$C_3$-Alkylen und
$Y_1$ und $Y_2$ unsubstituiertes $C_1$-$C_2$-Alkyl,
bedeuten,

(15) 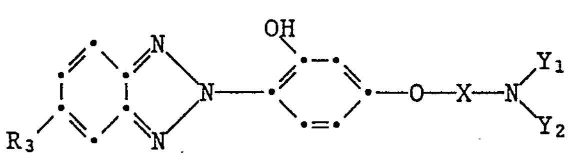

worin
$R_3$ Wasserstoff oder Chlor und
X $C_2$-$C_3$-Alkylen bedeuten und $Y_1$ und $Y_2$ die unter Formel (1) angegebene Bedeutung haben und

(16) 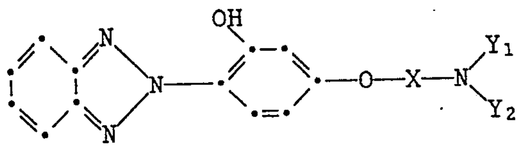

worin
X $C_2$-$C_3$-Alkylen und
$Y_1$ und $Y_2$ unsubstituiertes $C_1$-$C_2$-Alkyl,
bedeuten,
stellen bevorzugte Ausführungsformen der Verbindungen der Formel (12) dar.

Die Verbindungen der Formel (12) können dadurch hergestellt werden, dass man eine Verbindung der Formel

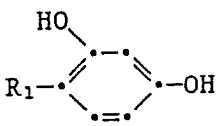

worin $R_1$ die oben angegebene Bedeutung hat mit einer Verbindung der Formel

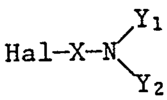

worin Hal Chlor oder Brom bedeutet und X, $Y_1$ und $Y_2$ die unter Formel (1) angegebene Bedeutung haben in Gegenwart einer Base, vorzugsweise Natrium-oder Kaliumhydroxid umsetzt.

Die Umsetzung wird bei einer Temperatur zwischen 60 und 120°C, vorzugsweise 30 und 60°C vorgenommen.

Die Verbindungen der Formel (12) können aber auch dadurch hergestellt werden, dass man eine Verbindung der Formel

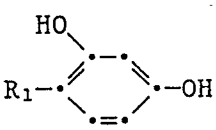

worin $R_1$ die oben angegebene Bedeutung hat,

a) mit einer Verbindung der Formel
Hal-X-OH
worin Hal Chlor oder Brom bedeutet und X die oben angegebene Bedeutung hat in Gegenwart einer Base umsetzt,

b) die erhaltene Verbindung der Formel

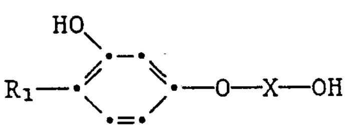

worin $R_1$ und X die oben angegebene Bedeutung haben, mit einem Halogenierungsmittel umsetzt und

c) die erhaltene Verbindung der Formel

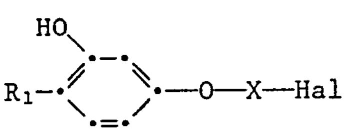

worin $R_1$, X und Hal die oben angegebenen Bedeutungen haben mit einem sekundären oder tertiären Amin der Formel

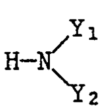

worin $Y_1$ und $Y_2$ die unter Formel (1) angegebene Bedeutung haben, umsetzt.

Als Halogenierungsmittel seien z.B. Halogenwasserstoffsäuren Hal-H, worin Hal Chlor oder Brom bedeutet, Thionylchlorid oder Thionylbromid genannt.

Die erste Stufe a) wird bei einer Temperatur zwischen 60 und 120°C, die zweite Stufe b) bei einer Temperatur zwischen 20 und 180°C und die dritte Stufe c) bei einer Temperatur zwischen 100 und 200°C vorgenommen.

Gegenstand der vorliegenden Erfindung sind demnach auch die Verfahren zur Herstellung von Verbindungen der Formel (12).

Die erfindungsgemässen kationischen Lichtschutzmittel werden beim Färben von basisch anfärbbaren, d.h. sauer modifizierten Polyamid- und/oder Polyesterfasermaterialien eingesetzt, um die gefärbten Fasern gegen fotochemische Zersetzung zu stabilisieren und die Lichtechtheit der Färbungen zu erhöhen.

Die Fasermaterialien, die in Gegenwart des neuen Lichtschutzmittels gefärbt werden können, sind flächenförmig vor allem Bodenbeläge, wie z.B. Teppiche. Sie können ausser aus den oben erwähnten sauer modifizierten Fasermaterialien auch noch aus Gemischen von unmodifizierten (basischen) und sauer modifizierten Polyamid- bzw. Polyesterfasermaterialien bestehen. Diese Fasermaterialien sind auch als "Differential dyeing"-Polyamide bzw. -Polyester bekannt und sind z.B. in W. Loy, Chemiefaserstoffe, Schiele und Schön, Berlin, 1978, Seiten 132-141 beschrieben.

Gegenstand der vorliegenden Erfindung ist demnach auch ein Verfahren zur Stabilisierung von basisch anfärbbaren Polyamid- und Polyesterfasermaterialien. Das Verfahren ist dadurch gekennzeichnet, dass man dieses Fasermaterial mit einer Färbeflotte behandelt, die neben einem Dispersionsfarbstoff bzw. kationischen Farbstoff noch eine Verbindung der Formel (1) enthält.

Die für das erfindungsgemässe Verfahren geeigneten kationischen Farbstoffe können verschiedenen Farbstoffklassen angehören. Insbesondere handelt es sich um die gebräuchlichen Salze, beispielsweise Chloride, Sulfate oder Metallhalogenide, wie z.B. Zinkchloriddoppelsalze von kationischen Farbstoffen, deren kationischer Charakter z.B. von einer Carbonium-, Oxonium-, Sulfonium- oder vor allem Ammoniumgruppe herrührt. Beispiele für solche chromophore Systeme sind Azofarbstoffe, vor allem Monoazo- oder Hydrazonfarbstoffe, Diphenylmethan-, Triphenylmethan-, Methin- oder Azomethinfarbstoffe, Cumarin-, Ketonimin-, Cyanin-, Azin-, Xanthen-, Oxazin- oder Thiazinfarbstoffe. Schliesslich können auch Farbsalze der Anthrachinonreihe mit externer Oniumgruppe, beispielsweise einer Alkylammonium- oder Cycloammoniumgruppe sowie Benzo-1,2-pyranfarbsalze, die Cycloammoniumgruppen enthalten, verwendet werden.

Die zu verwendenden Dispersionsfarbstoffe, die in Wasser nur sehr wenig löslich sind und in der Farbflotte zum grössten Teil in Form einer feinen Dispersion vorliegen, können den verschiedensten Farbstoffklassen angehören, beispielsweise den Acridon-, Azo-, Anthrachinon-, Cumarin-, Methin-, Perinon-, Naphthochinonimin-, Chinophthalon-, Styryl- oder Nitrofarbstoffen.

Es können auch Mischungen von kationischen oder Dispersionsfarbstoffen erfindungsgemäss eingesetzt werden.

Ein weiterer Gegenstand der vorliegenden Erfindung ist ein Verfahren zur Stabilisierung von Mischungen aus sauer und basisch färbbaren Polyamidfasermaterialien. Das Verfahren ist dadurch gekennzeichnet, dass man dieses Fasermaterial mit einer Flotte behandelt, die neben einem kationischen Farbstoff und einer Verbindung der Formel (1) noch einen Säurefarbstoff enthält.

Bei den Säurefarbstoffen handelt es sich beispielsweise um Salze metallfreier Mono-, Dis- oder Polyazofarbstoffe einschliesslich der Formazanfarbstoffe sowie der Anthrachinon-, Xanthen-, Nitro-, Triphenylmethan-und Naphthochinoniminfarbstoffe. Der saure Charakter dieser Farbstoffe wird durch saure, salzbildende Substituenten, wie Carbonsäuregruppen, Schwefelsäure- und Phosphonsäureestergruppen, Phosphonsäuregruppen oder Sulfonsäuregruppen bedingt. Diese Farbstoffe können im Molekül auch sogenannte reaktive Gruppierungen, welche mit dem zu färbenden Material eine kovalente Bindung eingehen, aufweisen. Bevorzugt sind saure, eine einzige Sulfonsäuregruppe aufweisende Farbstoffe.

Es können auch Mischungen der Säurefarbstoffe eingesetzt werden. Beispielsweise können Farbstoffmischungen von mindestens 2 oder 3 Säurefarbstoffen verwendet werden.

Ein weiterer Gegenstand der vorliegenden Erfindung ist ein Verfahren zur Stabilisierung von mit kationischen Farbstoffen gefärbten Polyacrylnitrilfasern. Das Verfahren ist dadurch gekennzeichnet, dass man das Fasermaterial mit einer Flotte behandelt, die neben einem migrierenden oder nicht-migrierenden kationischen Farbstoff noch eine Verbindung der Formel (1) enthält.

Als solche kationische Farbstoffe kommen die weiter oben erwähnten in Betracht.

Die Temperatur, bei der gefärbt wird, beträgt mindestens 70°C und in der Regel ist sie nicht höher als 106°C. Vorzugsweise liegt sie zwischen 80 und 130°C.

Die Menge der verwendeten Farbstoffe richtet sich nach der gewünschten Farbtiefe. Im allgemeinen haben sich Mengen von 0,001 bis 10 Gewichtsprozent, insbesondere 0,01 bis 5 Gewichtsprozent, bezogen auf das eingesetzte Fasermaterial, bewährt.

Als Fasermaterial sind synthetisches sauer modifiziertes Polyamid oder sauer modifiziertes Polyester, allein oder auch in Mischungen zu erwähnen. Als synthetisches sauer modifiziertes Polyamid kommt z.B. solches aus Adipinsäure und Hexamethylendiamin (Polyamid 6,6) aus ε-Caprolactam (Polyamid 6), aus ω-Aminoundecansäure (Polyamid 11), aus ω-Aminoönanthsäure (Polyamid 7), aus ω-Amino-perlargonsäure (Polyamid 8) oder aus Sebacinsäure und Hexamethylendiamin (Polyamid 6,10) in Betracht, das mit Carbonsäuren oder Sulfocarbonsäuren modifiziert worden ist.

Als sauer modifiziertes Polyester ist beispielsweise das Polykondensationsprodukt von Terephthalsäure oder Isophthalsäure, Ethylenglykol und 1,2- bzw. 1,3-Dihydroxy-3-(3-natriumsulfopropoxy)-propan, 2,3-Dimethylol-1-(natriumsulfopropoxy)-butan, 2,2-Bis-(3-natriumsulfopropoxyphenyl)- propan oder 3,5-Dicarboxybenzolsulfonsäure bzw. sulfonierter Terephthalsäure, sulfonierter 4-Methoxybenzolcarbonsäure oder sulfonierter Diphenyl-4,4′-dicarbonsäure.

Die zur Anwendung gelangenden Färbeflotten enthalten vorteilhafterweise Mineralsäuren, wie z.B. Schwefelsäure oder Phosphorsäure, oder organische Säuren, wie z.B. Ameisensäure, Essigsäure, Oxalsäure oder vorzugsweise Zitronensäure. Sie können auch Salze wie Ammoniumacetat, Ammoniumsulfat oder Natriumacetat enthalten. Die Säuren dienen vor allem zur Einstellung des pH-Wertes der Zubereitungen oder Flotten, der in der Regel 3 bis 7, vorzugsweise 3,5 bis 4,5 beträgt.

Zusätzlich zu den Lichtschutzmitteln, den Farbstoffen oder optischen Aufhellern können weitere in der Färbereitechnik übliche Hilfsmittel mitverwendet werden. Sie sind z.B. Dispergiermittel, Egalisiermittel, Elektrolyte, Netzmittel, Entschäumer, Schaumverhütungsmittel oder Verdicker.

Die zur Anwendung gelangenden Flotten können ausserdem noch photochemisch wirkende Antioxidantien wie Kupferkomplexe von Bisazomethinen enthalten. Diese Kupferkomplexe sind z.B. aus US-A-4,655,783 bekannt.

Die nachfolgenden Beispiele dienen zur Erläuterung der Erfindung. Darin sind die Teile Gewichtsteile und die Prozente Gewichtsprozente.

Beispiel 1:

20,5 g 2-(2′,4′-Dihydroxyphenyl)-4,6-diphenyl-1,3,5-triazin werden mit 120 ml Cyclohexanol auf 60° C erhitzt. Danach werden nacheinander 11,35 g 2-Diäthylamino-äthylchloridhydrochlorid und 7,4 g Natriummethylat (97 %) zugegeben. Die Mischung wird auf 120° C erhitzt und 2 Stunden bei dieser Temperatur gerührt. Anschliessend wird das Cyclohexanol im Vacuum abdestilliert, der Rückstand mit 50 ml Ethanol verrührt, abfiltriert, mit Ethanol und Wasser gewaschen und im Vacuum bei 60° C getrocknet. Man erhält 21 g Produkt der Formel

(100)
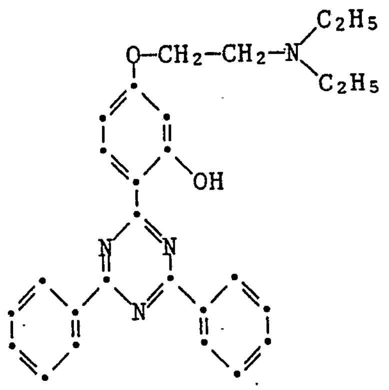

mit einem Schmelzpunkt von 94 - 95° C.

Verfährt man wie oben beschrieben, verwendet jedoch 22,4 g 2-(2′,4′-Dihydroxyphenyl)-4,6-bis-(2″,4″-dimethylphenyl)-1,3,5-triazin anstelle von 20,5 g 2-(2′,4′-Dihydroxyphenyl)-4,6-diphenyl-1,3,5-triazin dann erhält man die Verbindung der Formel

(101)
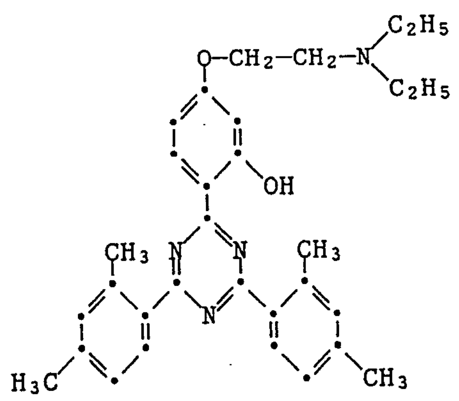

mit einem Schmelzpunkt von 112-113° C.

Verwendet man an Stelle der 11,35 g 2-Diäthylamino-äthylchloridhydrochlorid 10,91 g 3-Dimethylamino-1-propylchloridhydrochlorid, dann erhält man, bei sonst gleicher Arbeitsweise, die Verbindung der Formel

(102)

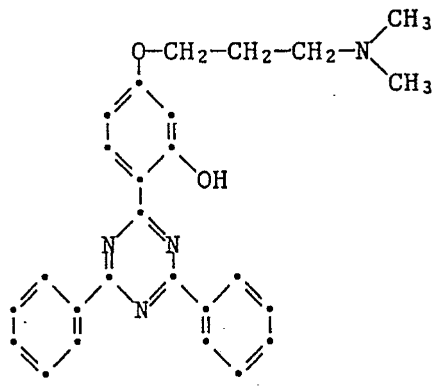

vom Schmelzpunkt 124 - 125°C.

Auf gleichem Weg lassen sich aus dem 2,4-Dihydroxybenzophenon und dem 2,4-Dihydroxyphenyl-benztriazol die Verbindungen

(103)

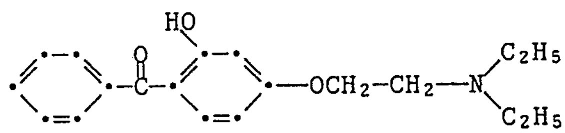

(fällt ölig an) und

(104)

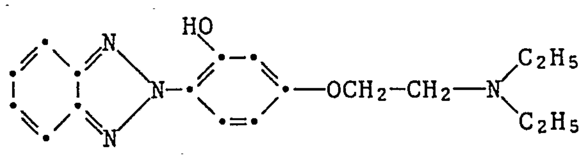

(fällt ölig an) herstellen.

Beispiel 2:

13.2 g der nach Beispiel 1 hergestellten Verbindung (100) werden in 100 ml Chlorbenzol gelöst und nach Zugabe von 4,16 g Dimethylsulfat auf 90°C erhitzt. Das dick ausgefallene Produkt wird noch 30 Minuten bei 90°C gerührt, danach auf 50°C abgekühlt, mit 50 ml Aceton versetzt und bei Raumtemperatur abfiltriert. Es wird mit Aceton gewaschen und dann getrocknet. Man erhält 16 g eines farblosen Produktes vom Schmelzpunkt 221 - 222°C der Formel

(200)

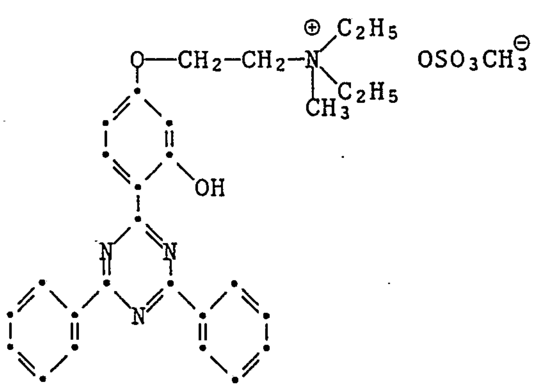

auf dem gleichen Weg lässt sich aus 14,4 g der Verbindung der Formel (101) die Verbindung der Formel

(201)

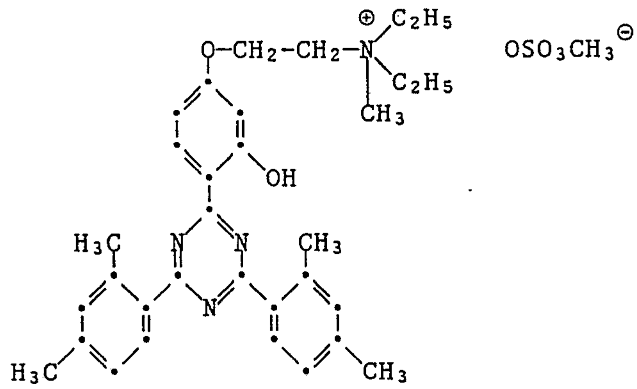

vom Schmelzpunkt 145-146°C herstellen.

Beispiel 3:

Verfährt man wie in Beispiel 2 beschrieben, verwendet jedoch an Stelle von 13,2 g der Verbindung der Formel (100) 9.8 g der Verbindung der Formel (103) oder 8,9 g der Verbindung der Formel (104) so erhält man die Verbindungen der Formeln

(300)

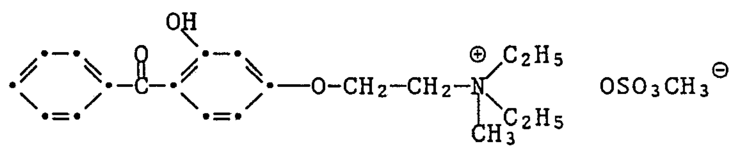

(fällt ölig an) und

(301)

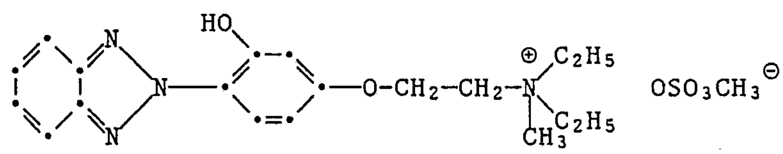

vom Schmelzpunkt 126 - 128°C.

Beispiel 4:

Verfährt man wie in Beispiel 2 beschrieben, verwendet jedoch an Stelle von 13,2 g der Verbindung der Formel (100) 12.8 g der Verbindung der Formel (102) so erhält man die Verbindung der Formel

(400)

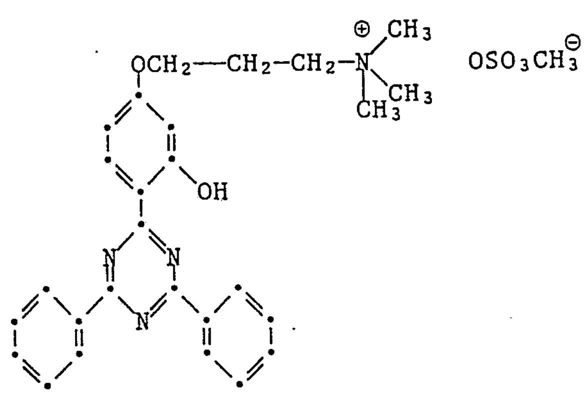

vom Schmelzpunkt 291 - 292°C.

Beispiel 5:

27,3 g 2-(2',4'-Dihydroxyphenyl)-4,6-diphenyl-1,3,5-triazin werden mit 275 ml Cyclohexanol auf 80°C erhitzt. Nach Zugabe von 4,96 g Natriummethylat wird auf 100°C erhitzt und anschiessend 13,5 g Bromethanol dazugegeben. Die gut rührbare Suspension wird danach auf 130-135°C erhitzt und 14 Stunden lang bei dieser Temperatur gerührt. Danach wird das Cyclohexanol im Vacuum abdestilliert und der Rückstand mit 50 ml Ethanol verrührt. Er wird abfiltriert, mit Ethanol und Wasser gewaschen und im Vakuum getrocknet. Man erhält

27,7 g Produkt der Formel

(500) 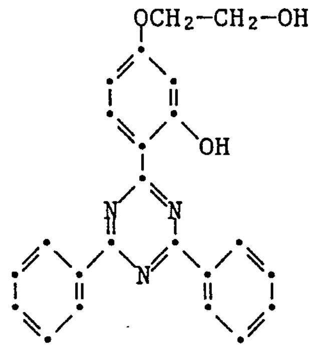

Die Verbindung kristallisiert aus Ethylenglykolmonomethyläther in farblosen Kristallen vom Smp. 230-231° C.

Beispiel 6:

46,3 g der in Beispiel 5 beschriebenen Verbindung werden mit 460 ml Chlorbenzol auf 100° C erhitzt. Bei 100-110° C werden danach in einer Stunde 15,7 Thionylchlorid eingetropft, wobei eine klare Lösung entsteht. Diese wird auf 120° C erhitzt und noch 3 Stunden bei dieser Temperatur gerührt. Anschliessend wird das Chlorbenzol im Vacuum abdestilliert und der Rückstand mit 50 ml Ethanol behandelt. Das Produkt wird abfiltriert, mit Ethanol gewaschen und bei 80° C im Vakuum getrocknet. Man erhält 48,2 g Produkt der Formel

(600) 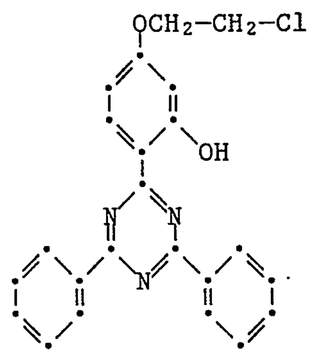

Die Verbindung kristallisiert aus Toluol in farblosen Kristallen vom Smp. 199-200° C.

Beispiel 7:

8,07 g der in Beispiel 6 beschriebenen Verbindung werden mit 25 ml Pyridin 14 Stunden lang auf Rückfluss erhitzt. Das schon in der Siedehitze ausgefallene Produkt wird bei 20° C abfiltriert, mit Pyridin und Aceton gewaschen und getrocknet. Man erhält 8,9 g der Verbindung der Formel

(700) 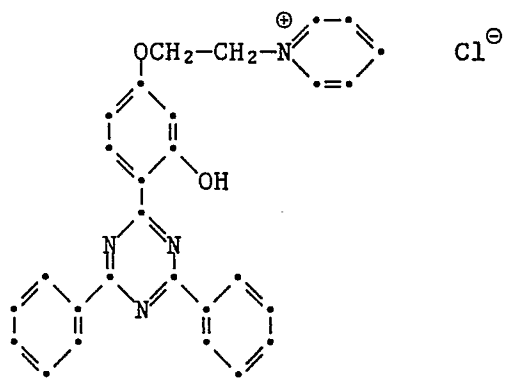

vom Smp. 244-245° C.

Beispiel 8:

7 Garnstränge von 10 g aus Polyamid 6,6-Stapelgarn werden in einen Färbeapparat (z.B. AHIBA®-Gerät) mit Flotten (Flottenverhältnis 1:50), die mit Essigsäure auf pH 4,5 eingestellt sind, behandelt und die noch

folgenden Farbstoffe und Zusätze enthalten (auf Fasermaterial berechnet):
Farbstoff der Formel

(800) 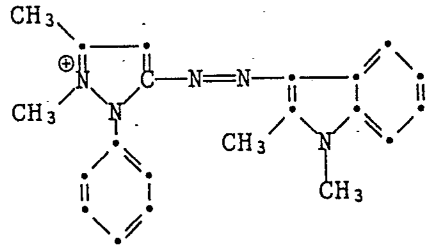 $ZnCl_3^{\ominus}$ (gelb)

Farbstoff der Formel

9 Teile

(801) 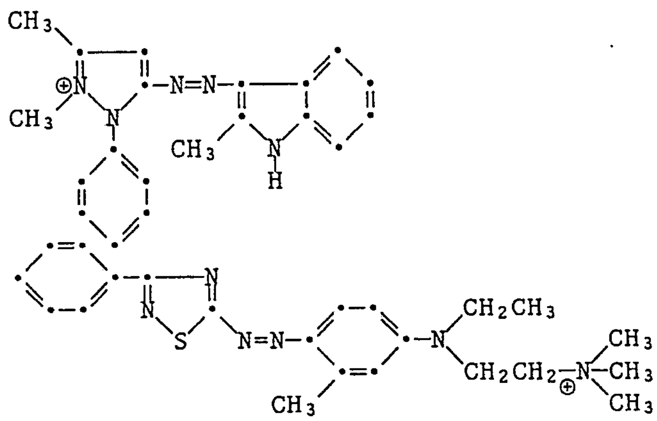 $OSO_3CH_3^{\ominus}$ $OSO_3CH_3^{\ominus}$ (rot)

36 Teile

Farbstoff der Formel

(802) 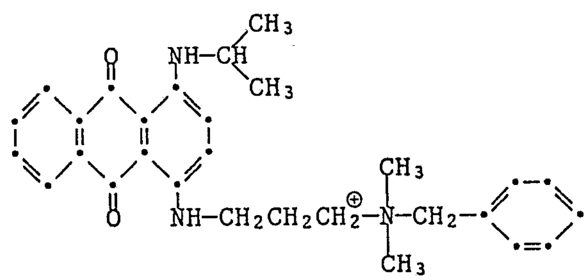 $Cl^{\ominus}$ (blau)

2 % eines nichtionischen Egalisiermittels

1. Flotte:
   0,06 % des Farbstoffes der Formel (800)
   0,06 % des Farbstoffes der Formel (801)
   0,02 % des Farbstoffes der Formel (802)
2. Flotte:
   wie Flotte 1. und zusätzlich 0,2 % der Verbindung der Formel (200).
3. Flotte:
   wie Flotte 1. und zusätzlich 0,5 % der Verbindung der Formel (200).
4. Flotte:
   wie Flotte 1. und zusätzlich
   1,0 % der Verbindung der Formel (200).
5. Flotte:
   wie Flotte 1 und zusätzlich
   0,05 % der Verbindung der Formel

(803) 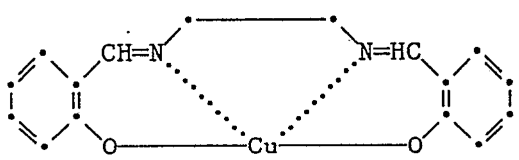

(in fein dispergierter Form)

6. Flotte:
wie Flotte 1. und zusätzlich
0,2 % der Verbindung der Formel

(804) 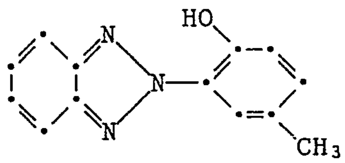

(in fein dispergierter Form)

7. Flotte:
wie Flotte 1, und zusätzlich
0,2 % der Verbindung der Formel (200) und
0,05 % der Verbindung der Formel (803) in fein dispergierter Form.

Man geht mit den Färbematerialien in die wie beschrieben vorbereitete Flotte von 40° C ein, behandelt 5 Minuten und heizt mit einer Geschwindigkeit von 1,5° C/Min. auf 95° C auf. Man färbt 30 Minuten lang bei dieser Temperatur, setzt 1 % Essigsäure (80 %), mit Wasser verdünnt, zu und färbt weitere 30 Minuten. Danach kühlt man ab, spült die Färbungen in kaltem Wasser, zentrifugiert und trocknet bei 80° C.

Die Färbungen werden anschliessend auf ihre Lichtechtheit hin geprüft nach folgenden Methoden
- Fakralicht gemäss DIN 75.202, Entwurf (Heissbelichtung) während 24 und 72 Stunden.
- Xenonlicht gemäss Schweizerischer Norm SN-ISO 105-B02 während 300 und 500 Stunden.

Die Resultate sind in der folgenden Tabelle I zusammengefasst.

Tabelle I

| Flotte Nr. | Lichtechtheit | | | | Reissfestigkeit/ Dehnung in % nach 72 h Fakra |
|---|---|---|---|---|---|
| | Fakra | | Xenon | | |
| | 24 h | 72 h | 300 h | 500 h | |
| 1 | < 4H | < 4H | 4 - 5 | 4 - 5 | zerfallen |
| 2 | 5 | 4H | 5 - 6 | 5 - 6 | 10.7 / 14.4 |
| 3 | 6 | 4H | 6 + | 6 + | 28.7 / 25.0 |
| 4 | 6 | 5H | 6 | 6 | 49.9 / 42.1 |
| 5 | 5G | 4HG | 4G | 4 - 5 G* | 86.6 / 79.3 |
| 6 | 6 | 4 - 5H | 6 | 6 | 22.8 / 27.2 |
| 7 | 6 - 7 | 6 + H | 5G | 5 - 6 | 99.0 / 88.1 |

Beispiel 9:

2 Garnstränge von 10 g aus Polyamid 6,6-Stapelgarn werden in einem Färbeapparat (z.B. AHIBA®-Gerät) mit Flotten (Flottenverhältnis 1:50) die mit Essigsäure auf pH 4,5 eingestellt sind, behandelt, die noch folgende Zusätze enthalten (auf Fasermaterial berechnet):

1. Flotte:
2,0 % eines nichtionischen Egalisiermittels
2. Flotte:
2,0 % eines nichtionischen Egalisiermittels und
0,2 % der Verbindung der Formel (200).

Man behandelt dann die Muster wie in Beispiel 8 beschrieben und prüft die Muster auf ihre fotochemische Stabilität hin.

Die Resultate sind in der folgenden Tabelle II zusammengefasst:

Tabelle II

| Flotte Nr. | Stabilität | | | |
|---|---|---|---|---|
| | Fakra | | Xenon | |
| | 24 h | 72 h | 300 h | 500 h |
| 1 | A[x] | B[xx] | keine optisch sichtbaren Unterschiede | |
| 2 | A[x] | C[xxx] | | |

[x]A wahrnehmbare Vergilbung aber Faser intakt,

[xx]B Faser vergilbt und Stabilität so beeinträchtigt, dass Faser bei geringster mechanischer Beanspruchung reisst,

[xxx]C Faser schwach vergilbt.

Beispiel 10:

Je 3 Muster eines Stapelgewebes von 10 g aus Dacron® 64 (150 g/m²) werden in einem Färbeapparat für Druckbomben bei einem Flottenverhältnis 1:20 gefärbt, wobei jeweils 3 Flotten angesetzt werden, die 1 g/l eines Egalisiermittels, 6 g/l Glaubersalz kalz., 2 % Essigsäure 80 % und 0,05 % des Gelbfarbstoffes der Formel

(1000) 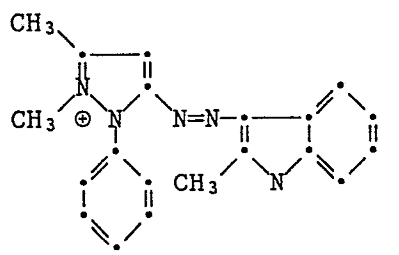

$Cl^{\ominus}$

$1/2\ SO_4^{\ominus\ominus}$

$CH_3SO_4^{\ominus}$

enthalten.

Flotte 1: enthält keine weiteren Zusätze.

Flotte 2: enthält zusätzlich 0,5 % der Verbindung der Formel (400)

Flotte 3: enthält zusätzlich 0,5 % der Verbindung der Formel (300) (alle % Angaben beziehen sich auf das Gewicht des Gewebes)

Man geht mit dem Gewebe bei 50° C in die vorbereitete Flotte ein und erhitzt in 30 Minuten auf 120° C; bei dieser Temperatur belässt man 60 Minuten. Danach kühlt man auf 50° C, spült es kalt und trocknet bei 80° C in einem Umluftofen.

Von den Färbungen werden die Lichtechtheiten nach "Xenon" (gemäss Schweizerischer Norm SN-ISO 105-BO2; Bewertung nach Blaumassstab 1 - 8) und "Fakra" (Heisslichtechtheit gemäss DIN 75 202, Bewertung nach Graumassstab 1 - 5) angefertigt. Das Ergebnis ist der folgenden Tabelle III zu entnehmen:

Tabelle III.

| Flotte Nr. | LICHTECHTHEITEN nach | | |
|---|---|---|---|
| | Xenon | Fakra 72 h | Fakra 144 h |
| 1 | 7 - 8 | 4 + | 3 - 4 H |
| 2 | 7 - 8 | 5 | 4 - 5 |
| 3 | 7 - 8 | 4 - 5 | 4 |

Beispiel 11:

Man verfährt wie in Beispiel 10 angegeben mit der Ausnahme, dass man 0,06 % des Rotfarbstoffes der Formel

(1100) 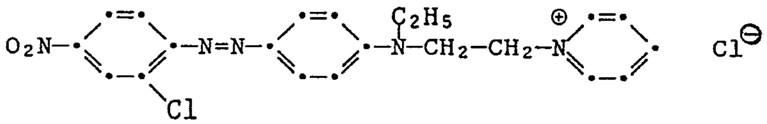

einsetzt.

Flotte 1: enthält keinen weiteren Zusatz.
Flotte 2: enthält 0,5 % der Verbindung der Formel (200).
Flotte 3: enthält 0,5 % der Verbindung der Formel (400).

Die Ergebnisse sind in der folgenden Tabelle IV zusammengefasst.

Tabelle IV

| Flotte Nr. | Lichtechtheiten nach | | |
|---|---|---|---|
| | Xenon | Fakra 72 h | Fakra 144 h |
| 1 | 4-5 | 2-3 H | 2 H |
| 2 | 6-7 | 4 | -3-4 H |
| 3 | 6 | 3-4 H | 3 H |

Beispiel 12:

Man verfährt wie in Beispiel 11 vermerkt mit dem Unterschied, dass man 0,05 % des Farbstoffes der Formel einsetzt:

(1200) 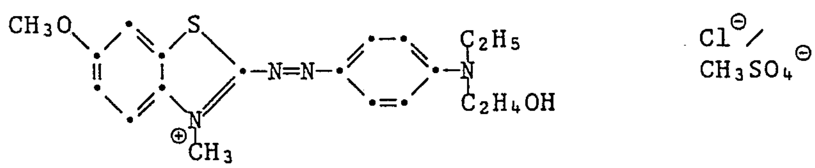

Flotte 1: enthält keine weiteren Zusätze.
Flotte 2: enthält 0,5 % der Verbindung der Formel (400).
Flotte 3: enthält 0,5 % der Verbindung der Formel (200).
Flotte 4: enthält 0,5 % der Verbindung der Formel (300).
Flotte 5: enthält 0,5 % der Verbindung der Formel (201).

Die Ergebnisse sind in der folgenden Tabelle V zusammengefasst.

Tabelle V

| Flotte Nr. | Lichtechtheiten* nach | | |
|---|---|---|---|
| | Xenon | Fakra 72 h | Fakra 144 h |
| 1 | 5 | 2 H | 1-2 H |
| 2 | 6+ | 3 H | -2-3 H |
| 3 | 6 | 2-3 H | 2 H |
| 4 | 5-6 | 2-3 H | 2 RH |
| 5 | 6 | 3 | -2-3 H |

* Xenon nach Blaumassstab 1-8

Fakra nach Graumassstab 1-5

**Patentansprüche**

1. Kationische Verbindungen der Formel

(1) 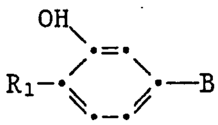

worin B eine Gruppe der Formel

(Ia) 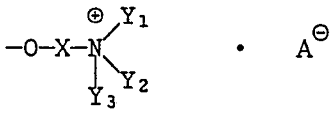 und

$R_1$ einen Rest der Formel

(2) 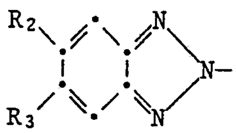 , (3) 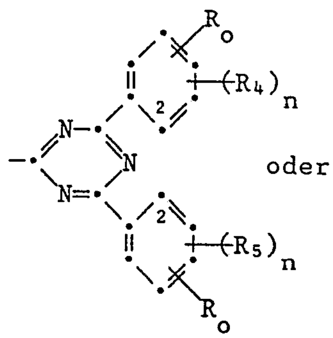 oder (4) 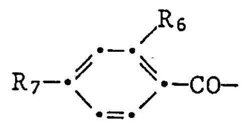

worin$R_0$ für Wasserstoff oder Hydroxy,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen oder wenn $R_0$ Hydroxy und n 1 sind auch die Gruppe der Formel (Ia),
$R_6$ für Wasserstoff, Hydroxy oder Carboxy,
$R_7$ für Wasserstoff, Hydroxy oder $C_1$-$C_4$-Alkoxy und
n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring,
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,
$Y_3$ Wasserstoff, unsubstituiertes oder durch Cyano, Hydroxy, $C_1$-$C_4$-Alkoxy, Phenyl oder $C_1$-$C_4$-Alkoxycarbonyl substituiertes $C_1$-$C_4$-Alkyl oder $C_3$-$C_4$-Alkenyl,
$Y_1$, $Y_2$ und $Y_3$ zusammen mit dem sie verbindenden N-Atom auch den Pyridin-oder Picolinring und
$A^{\ominus}$ ein farbloses Anion
bedeuten.

2. Verbindungen gemäss Anspruch 1, worin $Y_1$ und $Y_2$ zusammen mit dem sie verbindenden N-Atom einen Morpholin-, Pyrrolidin-, Piperidin- oder Hexamethyleniminring als 5- bis 7-gliedrigen heterocyclischen Ring bedeuten.

3. Verbindungen gemäss Anspruch 1 der Formel

(5)

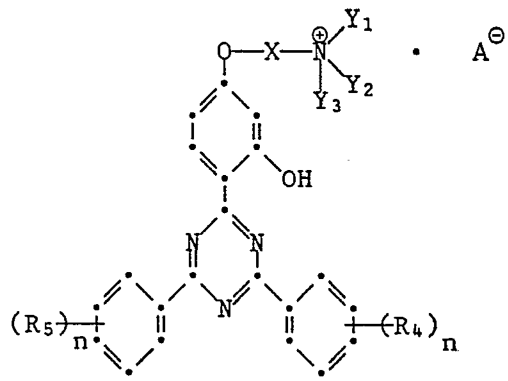

worin $R_4$ und $R_5$ Wasserstoff oder $C_1$-$C_4$-Alkyl, X $C_2$-$C_3$-Alkylen bedeuten und $Y_1$, $Y_2$, $Y_3$, n und $A^\ominus$ die in Anspruch 1 angegebenen Bedeutung haben.

4. Verbindungen gemäss Anspruch 3 der Formel

(6)

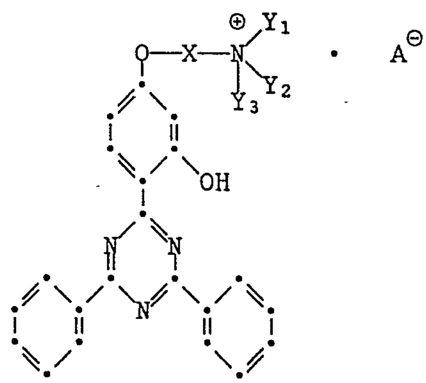

worin X $C_2$-$C_3$-Alkylen,
$Y_1$ und $Y_2$ unsubstituiertes $C_1$-$C_2$-Alkyl,
$Y_3$ Methyl oder Ethyl und
$A^\ominus$ $CH_3OSO_3^\ominus$ oder $C_2H_5OSO_3^\ominus$
bedeuten.

5. Verbindungen gemäss Anspruch 1 der Formel

(7)

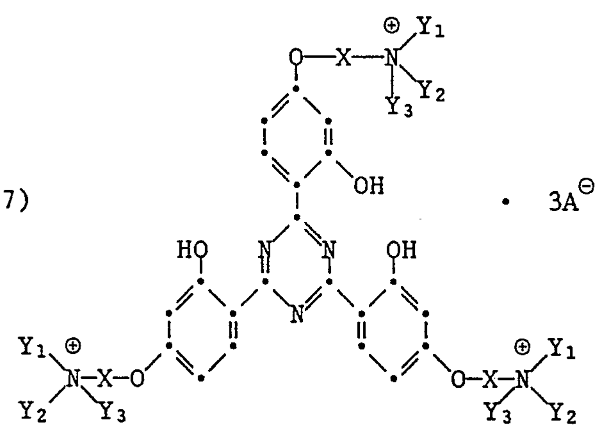

worin X, $Y_1$, $Y_2$, $Y_3$ und $A^\ominus$ die in Anspruch 1 angegebene Bedeutung haben.

6. Verbindungen gemäss Anspruch 1 der Formel

(8)

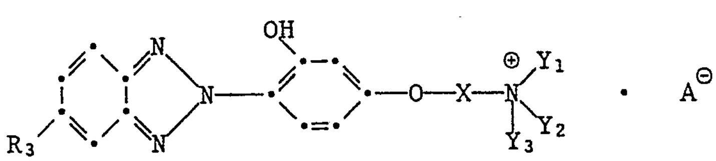

worin

$R_3$ Wasserstoff oder Chlor und
X $C_2$-$C_3$-Alkylen bedeuten und $Y_1$, $Y_2$, $Y_3$ und $A^{\ominus}$ die in Anspruch 1 angegebene Bedeutung haben.

7. Verbindungen gemäss Anspruch 6 der Formel

(9) 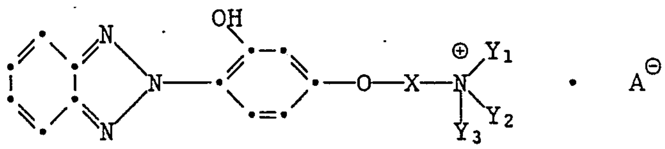

worin
X $C_2$-$C_3$-Alkylen,
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

8. Verbindungen gemäss Anspruch 1 der Formel

(10) 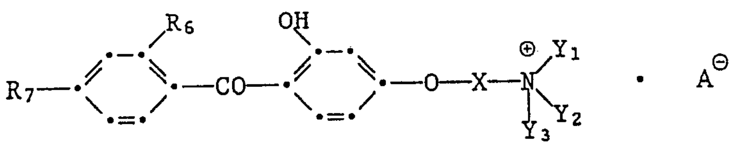

worin
X $C_2$-$C_3$-Alkylen bedeutet und $R_6$, $R_7$, $Y_1$, $Y_2$, $Y_3$ und $A^{\ominus}$ die in Anspruch 1 angegebene Bedeutung haben.

9. Verbindungen gemäss Anspruch 8 der Formel

(11) 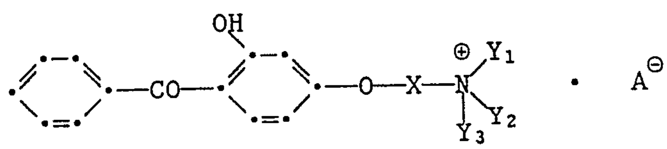

worin
X $C_2$-$C_3$-Alkylen
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

10. Verfahren zur Herstellung von Verbindungen der Formel

(1) 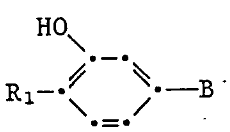

worin B eine Gruppe der Formel

(Ia) 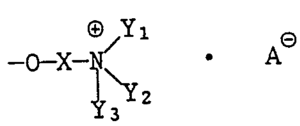 und

$R_1$ einen Rest der Formel

(2) 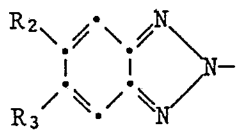 , (3) 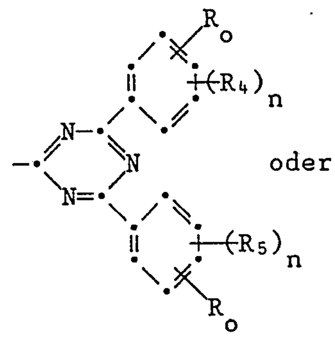 oder (4) 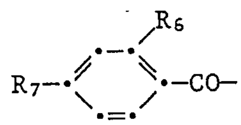

worin
$R_0$ für Wasserstoff oder Hydroxy,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen oder wenn $R_0$ Hydroxy und n 1 sind auch die Gruppe der Formel (Ia),
$R_6$ für Wasserstoff, Hydroxy oder Carboxy,
$R_7$ für Wasserstoff, Hydroxy oder $C_1$-$C_4$-Alkoxy und
n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring,
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,
$Y_3$ Wasserstoff, unsubstituiertes oder durch Cyano, Hydroxy, $C_1$-$C_4$-Alkoxy, Phenyl oder $C_1$-$C_4$-Alkoxycarbonyl substituiertes $C_1$-$C_4$-Alkyl oder $C_3$-$C_4$-Alkenyl,
$Y_1$, $Y_2$ und $Y_3$ zusammen mit dem sie verbindenden N-Atom auch den Pyridin-oder Picolinring und
$A^{\ominus}$ ein farbloses Anion
bedeuten, dadurch gekennzeichnet, dass man 1 Moläquivalent einer Verbindung der Formel

(12) 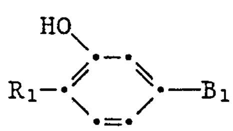

mit einem Moläquivalent eines Alkylierungsmittels bzw. einer Säure der Formel $Y_3$-A bei einer Temperatur von 0 - 180°C quaterniert bzw. protoniert, wobei in diesen Formeln $B_1$ die Gruppe der Formel

(Ib) 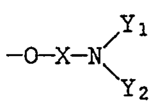

bedeutet und $R_1$, X, $Y_1$, $Y_2$, $Y_3$ und A die oben angegebene Bedeutung haben.

11. Verfahren gemäss Anspruch 10, dadurch gekennzeichnet, dass die Quaternierung bzw. Protonierung bei einer Temperatur von 30 - 140°C vorgenommen wird.

12. Verbindungen der Formel

(12) 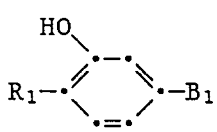

worin $B_1$ die Gruppe der Formel

(Ib) 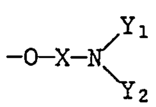 und

$R_1$ einen Rest der Formel

(2) 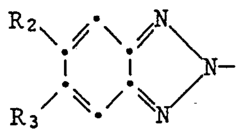 , (3) 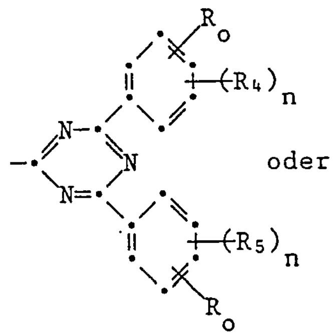 oder (4) 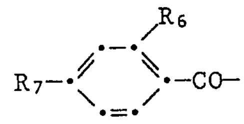

worin
$R_0$ für Wasserstoff oder Hydroxy,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen oder wenn $R_0$ Hydroxy und n 1 sind auch die Gruppe der Formel (Ib),
$R_6$ für Wasserstoff, Hydroxy oder Carboxy,
$R_7$ für Wasserstoff, Hydroxy oder $C_1$-$C_4$-Alkoxy und
n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring und
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,
bedeuten.

13. Verbindungen gemäss Anspruch 12, worin $Y_1$ und $Y_2$ zusammen mit dem sie verbindenden N-Atom einen Morpholin-, Pyrrolidin-, Piperidin- oder Hexamethyleniminring als 5- bis 7-gliedrigen heterocyclischen Ring bedeuten.

14. Verbindungen gemäss Anspruch 12 der Formel

(13) 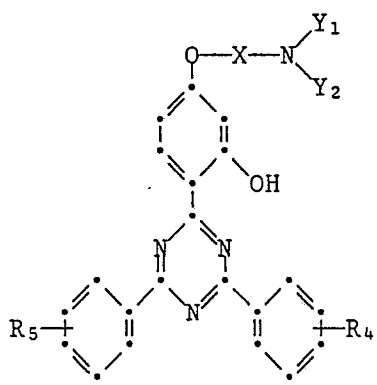

worin $R_4$ und $R_5$ Wasserstoff oder $C_1$-$C_4$-Alkyl, X $C_2$-$C_3$-Alkylen bedeuten und $Y_1$ und $Y_2$ die in Anspruch 12 angegebene Bedeutung haben.

15. Verbindungen gemäss Anspruch 14 der Formel

(14) 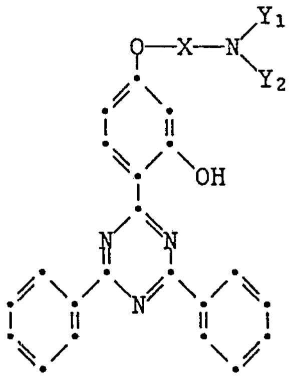

worin X $C_2$-$C_3$-Alkylen und
$Y_1$ und $Y_2$ unsubstituiertes $C_1$-$C_2$-Alkyl, bedeuten.

16. Verbindungen gemäss Anspruch 12 der Formel

(15) 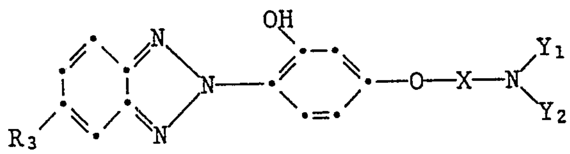

worin
$R_3$ Wasserstoff oder Chlor und
X $C_2$-$C_3$-Alkylen bedeuten und $Y_1$ und $Y_2$ die in Anspruch 12 angegebene Bedeutung haben.

17. Verbindungen gemäss Anspruch 16 der Formel

(16) 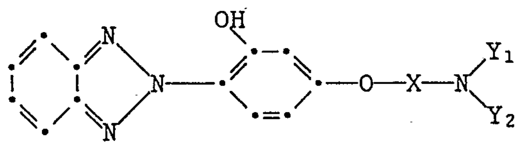

worin
X $C_2$-$C_3$-Alkylen und
$Y_1$ und $Y_2$ unsubstituiertes $C_1$-$C_2$-Alkyl,
bedeuten.

18. Verbindungen gemäss Anspruch 12 der Formel

(17) 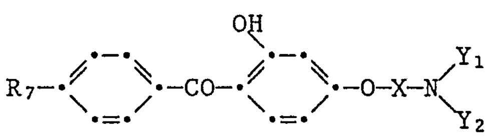

worin
X $C_2$-$C_3$-Alkylen bedeutet und $R_6$, $R_7$, $Y_1$ und $Y_2$ die in Anspruch 12 angegebene Bedeutung haben.

19. Verbindungen gemäss Anspruch 18 der Formel

(18) 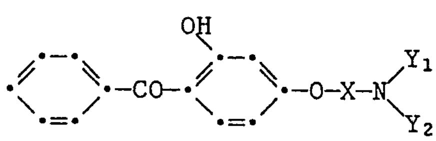

worin
X $C_2$-$C_3$-Alkylen und
$Y_1$ und $Y_2$ unsubstituiertes $C_1$-$C_2$-Alkyl
bedeuten.

20. Verfahren zur Herstellung von Verbindungen der Formel

(12) 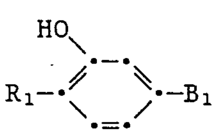

worin $B_1$ die Gruppe der Formel

(Ib) 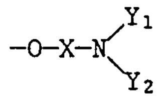 und

$R_1$ einen Rest der Formel

(2) 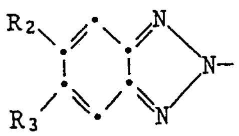 , (3) 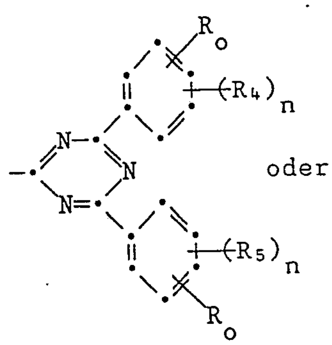 oder (4) 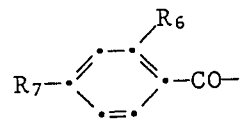

worin
$R_0$ für Wasserstoff oder Hydroxyd,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen, oder wenn $R_0$ Hydroxy und n 1 sind auch die Gruppe der Formel (Ib),
$R_6$ für Wasserstoff, Hydroxy oder Carboxy,
$R_7$ für Wasserstoff, Hydroxy oder $C_1$-$C_4$-Alkoxy und
n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring und
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,
bedeuten, dadurch gekennzeichnet, dass man

A) eine Verbindung der Formel

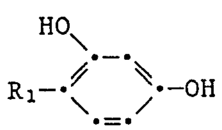

worin $R_1$ die oben angegebene Bedeutung hat mit einer Verbindung der Formel

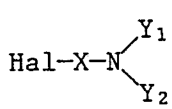

worin Hal Chlor oder Brom bedeutet und X, $Y_1$ und $Y_2$ die oben angegebene Bedeutung haben in Gegenwart einer Base umsetzt, oder

B) dass man eine Verbindung der Formel

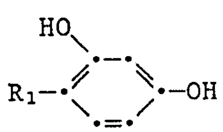

worin $R_1$ die oben angegebene Bedeutung hat
a) mit einer Verbindung der Formel
Hal-X-OH

worin Hal Chlor oder Brom bedeutet und X die oben angegebene Bedeutung hat in Gegenwart einer Base umsetzt,

b) die erhaltene Verbindung der Formel

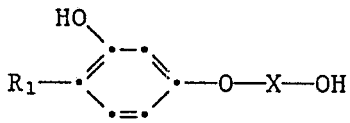

worin $R_1$ und X die oben angegebene Bedeutungen haben, mit einem Halogenierungsmittel umsetzt und

c) die erhaltene Verbindung der Formel

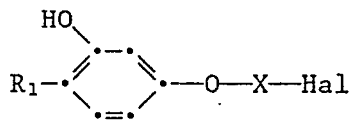

worin $R_1$, X und Hal die oben angegebenen Bedeutung haben mit einem sekundären oder tertiären Amin der Formel

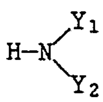

worin $Y_1$ und $Y_2$ die weiter oben angegebene Bedeutung haben, umsetzt.

21. Verfahren zur fotochemischen Stabilisierung von basisch anfärbbaren Polyamid- und Polyesterfasermaterialien mit Lichtschutzmitteln, dadurch gekennzeichnet, dass man das basisch anfärbbare Fasermaterial mit einer Färbeflotte behandelt, die neben einem Dispersionsfarbstoff bzw. kationischen Farbstoff eine Verbindung der Formel

(1) 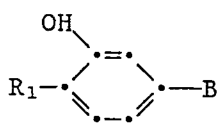

worin B eine Gruppe der Formel

(Ia) 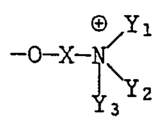 $\cdot\ A^{\ominus}$ und

$R_1$ einen Rest der Formel

(2) 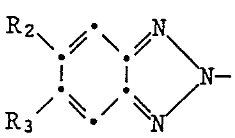 , (3) 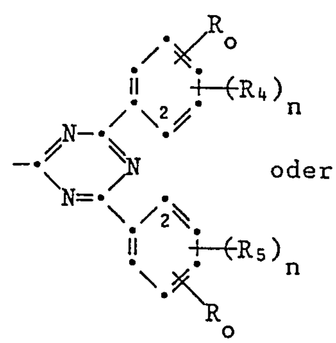 oder (4) 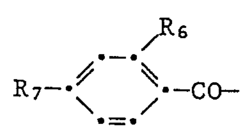

$R_0$ für Wasserstoff oder Hydroxy,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen oder wenn $R_0$ Hydroxy und n 1 sind auch die Gruppe der Formel (Ia),
$R_6$ für Wasserstoff, Hydroxy oder Carboxy,
$R_7$ für Wasserstoff, Hydroxy oder $C_1$-$C_4$-Alkoxy und

n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring,
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,
$Y_3$ Wasserstoff, unsubstituiertes oder durch Cyano, Hydroxy, $C_1$-$C_4$-Alkoxy, Phenyl oder $C_1$-$C_4$-Alkoxycarbonyl substituiertes $C_1$-$C_4$-Alkyl oder $C_3$-$C_4$-Alkenyl,
$Y_1$, $Y_2$ und $Y_3$ zusammen mit dem sie verbindenden N-Atom auch den Pyridin-oder Picolinring und
$A^{\ominus}$ ein farbloses Anion
bedeuten.

22. Verfahren gemäss Anspruch 21 zur Stabilisierung von basisch anfärbbaren Polyamidfasermaterialien, dadurch gekennzeichnet, dass die Färbeflotte noch ein Kupferkomplex eines Bisazomethins enthält.

23. Verfahren zur fotochemischen Stabilisierung von mit kationischen Farbstoffen anfärbbaren Polyacrylnitrilfasermaterialien, dadurch gekennzeichnet, dass man diese Materialien mit Flotten färbt, welche neben einem kationischen Farbstoff noch eine Verbindung der in Anspruch 1 definierten Formel (1) enthält.

24. Verwendung der in Anspruch 1 definierten kationischen Verbindungen zur fotochemischen Stabilisierung von basisch anfärbbaren Polyamid-, Polyacrylnitril- und Polyesterfasermaterialien.

25. Das gemäss dem Verfahren nach einem der Ansprüche 21 bis 23 stabilisierte basisch anfärbbare Polyamid-, Polyacrylnitril- und Polyesterfasermaterial.

**Patentansprüche für folgenden Vertragsstaat:ES**

1. Verfahren zur fotochemischen Stabilisierung von basisch anfärbbaren Polyamid- und Polyesterfasermaterialien mit Lichtschutzmitteln, dadurch gekennzeichnet, dass man das basisch anfärbbare Fasermaterial mit einer Färbeflotte behandelt, die neben einem Dispersionsfarbstoff bzw. kationischen Farbstoff eine Verbindung der Formel

(1) 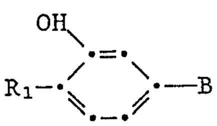

worin B eine Gruppe der Formel

(Ia) 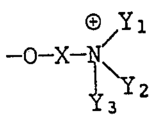 $\cdot$ $A^{\ominus}$ und

$R_1$ einen Rest der Formel

(2) 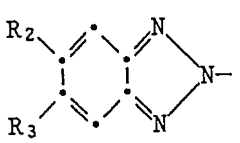 , (3) 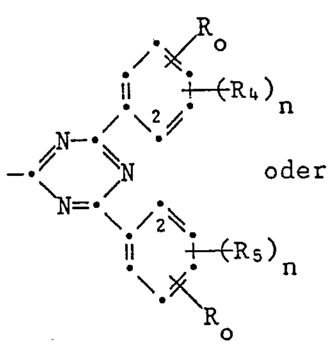 oder (4) 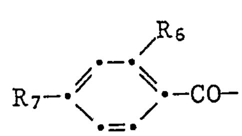

$R_0$ für Wasserstoff oder Hydroxy,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen oder wenn $R_0$ Hydroxy und n 1 sind auch die Gruppe der Formel (Ia),
$R_6$ für Wasserstoff, Hydroxy oder Carboxy,

$R_7$ für Wasserstoff, Hydroxy oder $C_1$-$C_4$-Alkoxy und
n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring,
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,
$Y_3$ Wasserstoff, unsubstituiertes oder durch Cyano, Hydroxy, $C_1$-$C_4$-Alkoxy, Phenyl oder $C_1$-$C_4$-Alkoxycarbonyl substituiertes $C_1$-$C_4$-Alkyl oder $C_3$-$C_4$-Alkenyl,
$Y_1$, $Y_2$ und $Y_3$ zusammen mit dem sie verbindenden N-Atom auch den Pyridin-oder Picolinring und
$A^{\ominus}$ ein farblose Anion
bedeuten.

2. Verfahren gemäss Anspruch 1 zur Stabilisierung von basisch anfärbbaren Polyamidfasermaterialien, dadurch gekennzeichnet, dass die Färbeflotte noch ein Kupferkomplex eines Bisazomethins enthält.

3. Verfahren gemäss Anspruch 1, worin Verbindungen der Formel

(5) 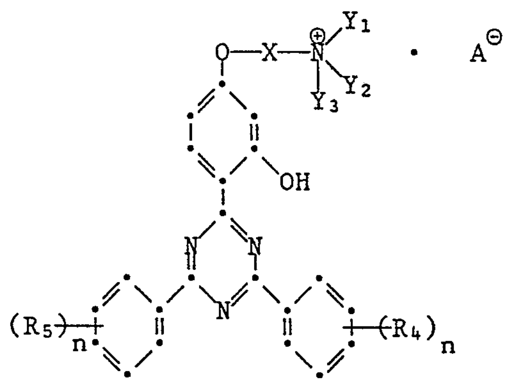

verwendet werden, worin $R_4$ und $R_5$ Wasserstoff oder $C_1$-$C_4$-Alkyl, X $C_2$-$C_3$-Alkylen bedeuten und $Y_1$, $Y_2$, $Y_3$, n und $A^{\ominus}$ die in Anspruch 1 angegebene Bedeutung haben.

4. Verfahren gemäss Anspruch 3, worin Verbindungen der Formel

(6) 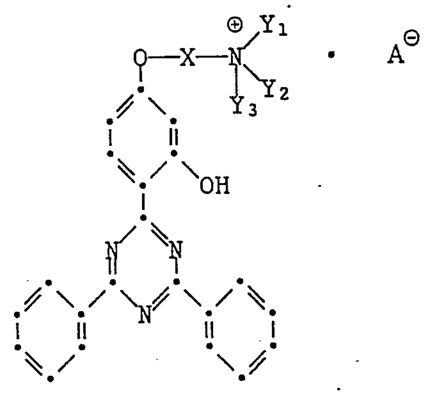

verwendet werden, worin X $C_2$-$C_3$-Alkylen,
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

5. Verfahren gemäss Anspruch 1, worin Verbindungen der Formel

(7) 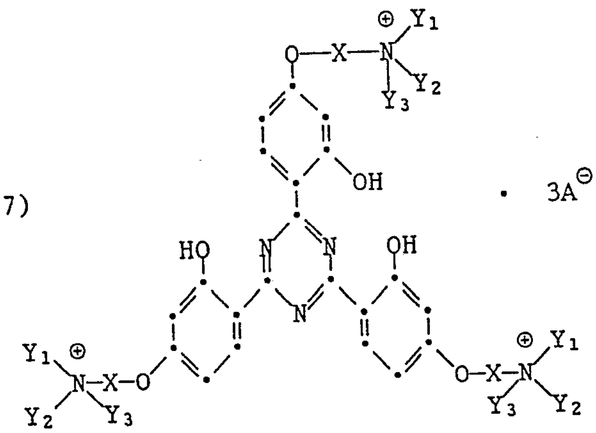

verwendet werden, worin X, $Y_1$, $Y_2$, $Y_3$ und $A^{\ominus}$ die in Anspruch 1 angegebene Bedeutung haben.

6. Verfahren gemäss Anspruch 1, worin Verbindungen der Formel

(8) 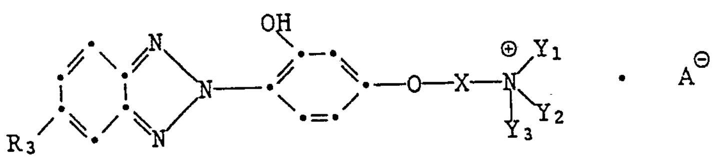

verwendet werden, worin
$R_3$ Wasserstoff oder Chlor und
X $C_2$-$C_3$-Alkylen bedeuten und $Y_1$, $Y_2$, $Y_3$ und $A^{\ominus}$ die in Anspruch 1 angegebene Bedeutung haben.

7. Verfahren gemäss Anspruch 6, worin Verbindungen der Formel

(9) 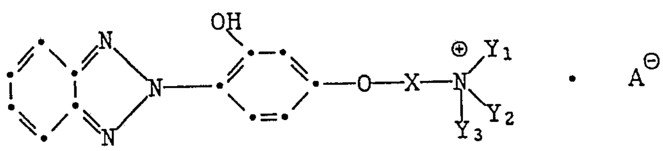

verwendet werden, worin
X $C_2$-$C_3$-Alkylen,
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

8. Verfahren gemäss Anspruch 1, worin Verbindungen der Formel

(10) 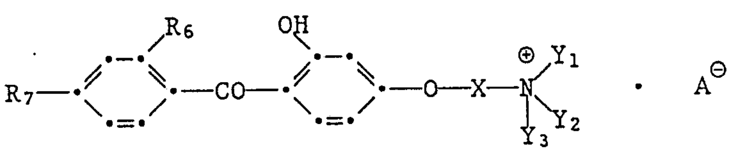

verwendet werden, worin
X $C_2$-$C_3$-Alkylen bedeutet und $R_6$, $R_7$, $Y_1$, $Y_2$, $Y_3$ und $A^{\ominus}$ die in Anspruch 1 angegebene Bedeutung haben.

9. Verfahren gemäss Anspruch 8, worin Verbindungen der Formel

(11) 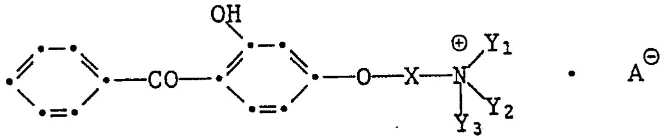

verwendet werden, worin
X $C_2$-$C_3$-Alkylen
$Y_1$ und $Y_2$ unsubstituiertes $C_1$-$C_2$-Alkyl,
$Y_3$ Methyl oder Ethyl und
$A^{\ominus}$ $CH_3OSO_3^{\ominus}$ oder $C_2H_5OSO_3^{\ominus}$
bedeuten.

10. Verfahren gemäss Anspruch 1, worin Verbindungen verwendet werden, worin $Y_1$ und $Y_2$ zusammen

mit dem sie verbindenden N-Atom einen Morpholin-, Pyrrolidin-, Piperidin- oder Hexamethyleniminring als 5- bis 7-gliedrigen heterocyclischen Ring bedeuten.

11. Verfahren zur Herstellung von Verbindungen der Formel

(1) 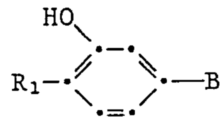

worin B eine Gruppe der Formel

(Ia) 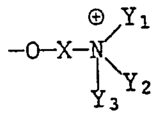 • $A^{\ominus}$ und

$R_1$ einen Rest der Formel

(2) 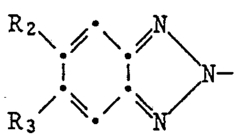 , (3) 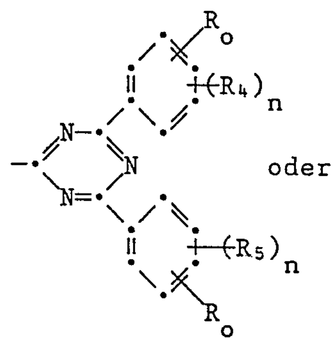 oder (4) 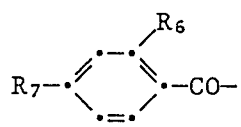

worin

$R_0$ für Wasserstoff oder Hydroxy,

$R_2$ für Wasserstoff, Halogen, $C_1$-$C_4$-Alkyl, $C_1$-$C_4$-Alkoxy, $C_2$-$C_9$-Alkoxycarbonyl oder Carboxy,

$R_3$ für Wasserstoff oder Halogen,

$R_4$ und $R_5$ unabhängig voneinander für Wasserstoff, $C_1$-$C_4$-Alkyl, $C_1$-$C_4$-Alkoxy oder Halogen oder wenn $R_0$ Hydroxy und n 1 sind auch die Gruppe der Formel (Ia),

$R_6$ für Wasserstoff, Hydroxy oder Carboxy,

$R_7$ für Wasserstoff, Hydroxy oder $C_1$-$C_4$-Alkoxy und

n für die Zahl 1 oder 2

stehen,

X $C_2$-$C_8$-Alkylen,

$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring,

$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring,

$Y_3$ Wasserstoff, unsubstituiertes oder durch Cyano, Hydroxy, $C_1$-$C_4$-Alkoxy, Phenyl oder $C_1$-$C_4$-Alkoxycarbonyl substituiertes $C_1$-$C_4$-Alkyl oder $C_3$-$C_4$-Alkenyl,

$Y_1$, $Y_2$ und $Y_3$ zusammen mit dem sie verbindenden N-Atom auch den Pyridin-oder Picolinring und

$A^{\ominus}$ ein farbloses Anion

bedeuten, dadurch gekennzeichnet, dass man 1 Moläquivalent einer Verbindung der Formel

(12) 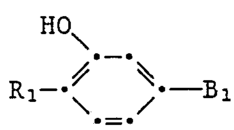

mit einem Moläquivalent eines Alkylierungsmittels bzw. einer Säure der Formel $Y_3$-A bei einer Temperatur von 0 - 180° C quaterniert bzw. protoniert, wobei in diesen Formeln $B_1$ die Gruppe der Formel

(Ib) 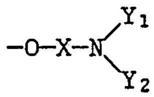

bedeutet und $R_1$, X, $Y_1$, $Y_2$, $Y_3$ und A die oben angegebene Bedeutung haben.

12. Verfahren gemäss Anspruch 11, dadurch gekennzeichnet, dass die Quaternierung bzw. Protonierung bei einer Temperatur von 30 - 140° C vorgenommen wird.

13. Verfahren zur Herstellung von Verbindungen der Formel

(12) 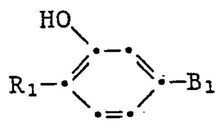

worin $B_1$ die Gruppe der Formel

(Ib) 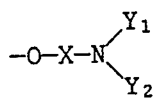 und

$R_1$ einen Rest der Formel

(2) 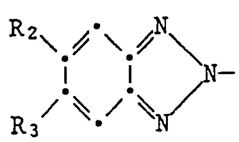 , (3) 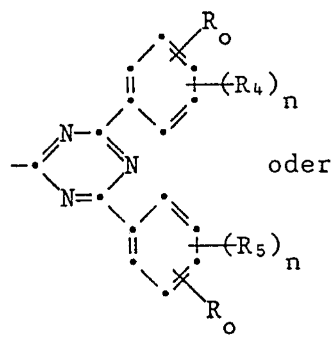 oder (4) 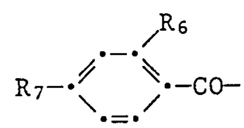

worin

$R_0$ für Wasserstoff oder Hydroxyd,
$R_2$ für Wasserstoff, Halogen, $C_1$-$C_4$-Alkyl, $C_1$-$C_4$-Alkoxy, $C_2$-$C_9$-Alkoxycarbonyl oder Carboxy,
$R_3$ für Wasserstoff oder Halogen,
$R_4$ und $R_5$ unabhängig voneinander für Wasserstoff, $C_1$-$C_4$-Alkyl, $C_1$-$C_4$-Alkoxy oder Halogen, oder wenn $R_0$ Hydroxy und n 1 sind auch die Gruppe der Formel (Ib),
$R_6$ für Wasserstoff, Hydroxy oder Carboxy,
$R_7$ für Wasserstoff, Hydroxy oder $C_1$-$C_4$-Alkoxy und
n für die Zahl 1 oder 2
stehen,
X $C_2$-$C_8$-Alkylen,
$Y_1$ unsubstituiertes oder durch Halogen, Cyano, Hydroxy oder $C_1$-$C_4$-Alkoxy substituiertes $C_1$-$C_4$-Alkyl oder zusammen mit $Y_2$ und dem sie verbindenden N-Atom einen 5- bis 7-gliedrigen heterocyclischen Ring und
$Y_2$ unsubstituiertes oder durch Halogen, Cyano, Hydroxy oder $C_1$-$C_4$-Alkoxy substituiertes $C_1$-$C_4$-Alkyl oder zusammen mit $Y_1$ und dem sie verbindenden N-Atom einen 5- bis 7-gliedrigen heterocyclischen Ring, bedeuten, dadurch gekennzeichnet, dass man

A) eine Verbindung der Formel

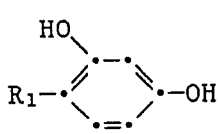

worin $R_1$ die oben angegebene Bedeutung hat mit einer Verbindung der Formel

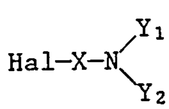

worin Hal Chlor oder Brom bedeutet und X, $Y_1$ und $Y_2$ die oben angegebene Bedeutung haben in Gegenwart einer Base umsetzt, oder

B) dass man eine Verbindung der Formel

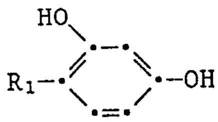

worin $R_1$ die oben angegebene Bedeutung hat

a) mit einer Verbindung der Formel

Hal-X-OH

worin Hal Chlor oder Brom bedeutet und X die oben angegebene Bedeutung hat in Gegenwart einer Base umsetzt,

b) die erhaltene Verbindung der Formel

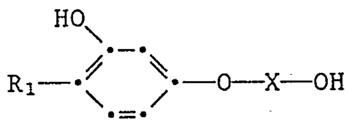

worin $R_1$ und X die oben angegebene Bedeutung haben, mit einem Halogenierungsmittel umsetzt und

c) die erhaltene Verbindung der Formel

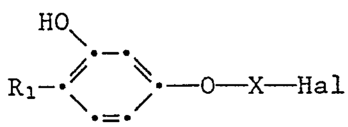

worin $R_1$, X und Hal die oben angegebenen Bedeutungen haben mit einem sekundären oder tertiären Amin der Formel

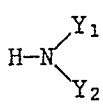

worin $Y_1$ und $Y_2$ die weiter oben angegebene Bedeutung haben, umsetzt.

14. Verfahren gemäss Anspruch 13 zur Herstellung von Verbindungen der Formel

(13) 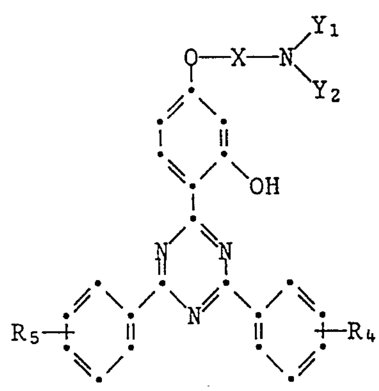

worin $R_4$ und $R_5$ Wasserstoff oder $C_1$-$C_4$-Alkyl, X $C_2$-$C_3$-Alkylen bedeuten und $Y_1$ und $Y_2$ die in Anspruch 13 angegebene Bedeutung haben.

15. Verfahren gemäss Anspruch 14 zur Herstellung von Verbindungen der Formel

(14) 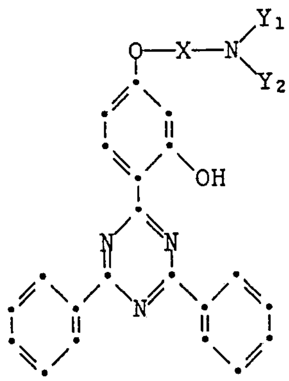

worin X $C_2$-$C_3$-Alkylen und
$Y_1$ und $Y_2$ unsubstituiertes $C_1$-$C_2$-Alkyl,
bedeuten.

16. Verfahren gemäss Anspruch 13 zur Herstellung von Verbindungen der Formel

(15) 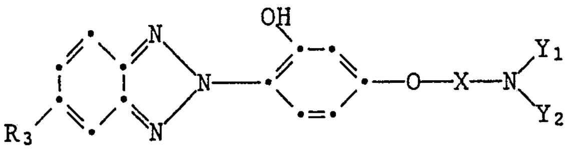

worin
$R_3$ Wasserstoff oder Chlor und
X $C_2$-$C_3$-Alkylen bedeuten und $Y_1$ und $Y_2$ die in Anspruch 12 angegebene Bedeutung haben.

17. Verfahren gemäss Anspruch 16 zur Herstellung von Verbindungen der Formel

(16) 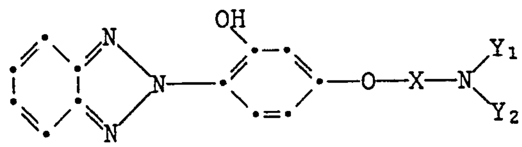

worin
X $C_2$-$C_3$-Alkylen und
$Y_1$ und $Y_2$ unsubstituiertes $C_1$-$C_2$-Alkyl,
bedeuten.

18. Verfahren gemäss Anspruch 13 zur Herstellung von Verbindungen der Formel

(17) 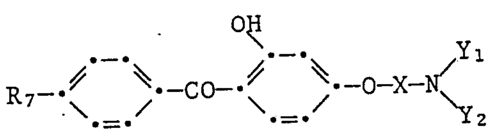

worin
X $C_2$-$C_3$-Alkylen bedeutet und $R_6$, $R_7$, $Y_1$ und $Y_2$ die in Anspruch 12 angegebene Bedeutung haben.

19. Verfahren gemäss Anspruch 18 zur Herstellung von Verbindungen der Formel

(18) 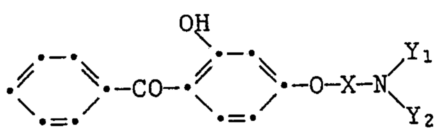

worin
X $C_2$-$C_3$-Alkylen und
$Y_1$ und $Y_2$ unsubstituiertes $C_1$-$C_2$-Alkyl
bedeuten.

20. Verfahren zur fotochemischen Stabilisierung von mit kationischen Farbstoffen anfärbbaren Polyacrylnitrilfasermaterialien, dadurch gekennzeichnet, dass man diese Materialien mit Flotten färbt, welche neben einem kationischen Farbstoff noch eine Verbindung der in Anspruch 1 definierten Formel (1) enthält.